# EUROPÄISCHE PATENTSCHRIFT

(11) **EP 0 790 249 B1**
(45) Veröffentlichungstag und Bekanntmachung des Hinweises auf die Patenterteilung: **11.04.2001**
(21) Anmeldenummer: 97101773.6
(22) Anmeldetag: 05.02.1997
(51) Int. Cl.: C07D 453/02, C07D 453/04

(54) **Enantiomerenreine Chinuklidinderivate und Verfahren zu ihrer Herstellung**
Enantiopure quinuclidine derivatives and process for their preparation
Enantiomères purs de dérivés de la quinuclidine et procédé pour leur préparation

(30) Priorität: 07.02.1996 DE 19604395
(43) Veröffentlichungstag der Anmeldung: 20.08.1997
(73) Patentinhaber: Buchler GmbH, 38110 Braunschweig (DE)
(72) Erfinder: Hoffmann, H.M.R. Prof. Institut für organ. Chemie, 30167 Hannover (DE)
(74) Vertreter: Gramm, Werner, Prof. Dipl.-Ing.

(56) Entgegenhaltungen:
- FR-A- 2 332 278
- FR-A- 2 332 279
- CHEMICAL ABSTRACTS, vol. 125, no. 15, 7.Oktober 1996 Columbus, Ohio, US; abstract no. 196082v, XP002030477 & SYNLETT, Nr. 7, 1996, Seiten 690-692, H.M.R. HOFFMANN ET AL.:
- TETRAHEDRON LETTERS, Nr. 29, 1977, OXFORD GB, Seiten 2471-2474, XP000673062 G.A. EPLING ET AL.:
- CHEMICAL ABSTRACTS, vol. 118, no. 19, 10.Mai 1993 Columbus, Ohio, US; abstract no. 192066a, XP002030478 & ZH. OBSHCH. KHIM., Bd. 62, Nr. 4, 1992, Seiten 923-927, A.M. GAZALIEV ET AL.:
- CHEMICAL ABSTRACTS, vol. 108, no. 19, 9.Mai 1988 Columbus, Ohio, US; abstract no. 167740b, XP002030479 & TETRAHEDRON, Bd. 43, Nr. 5, 1987, OXFORD GB, Seiten 911-922, Y. YANUKA ET AL.:

## Beschreibung

Die Erfindung betrifft neue enantiomerenreine Chinuklidinderivate, ein Verfahren zu ihrer Herstellung sowie deren Verwendung als Zwischenprodukte in der Pharmasynthese.

Muscarin erregt die postganglionären parasympatischen Rezeptoren und hat deshalb für die experimentelle Pharmakologie eine große Bedeutung. Therapeutisch wird es jedoch nicht angewendet. Chinuklidinderivate sind Muscarinagonisten, von denen angenommen wird, daß sie ein wirksames Mittel zur Behandlung der Alzheimer Krankheit sein können.

Tetrahedron Let. Nr. 31 (1978), S.2779-2782 beschreibt die Umwandlung von Chinin in Chinidin durch Oxydation. FR 1 472 016 bezieht sich auf ein Verfahren zur Herstellung von Verbindungen durch Substitution am Chinuklidin-Kern. Das Chinuklidin als solches wird dabei erhalten, es erfolgt keine Abspaltung des Chinolinanteils. Tetrahedron Let. 31, Nr. 16 (1990), S.2281-2282 beschreibt die gleichzeitige Umsetzung von Chinin mit einem Reduktionsmittel (Zinkstaub) und einem Oxidationsmittel (Luft), auch hier kommt es nicht zu einer Chinolinabspaltung. Tetrahedron Let. 35, Nr. 8 (1994), S.1137-1140 schlägt die Verbindung eines Chinuklidinderivats in Enonform in das entsprechende Alkanol durch Reduktion mit LiAlH₄ in THF vor, um eine stereoselektive Reduktion durchzuführen. Tetrahedron Let. Nr. 29 (1977), S.2471-2474 beschreibt die Fotolyse von Chinaalkaloiden, wobei Epimerengemische von Aldehyden anfallen, die nicht getrennt werden können und sich spontan ineinander umwandeln. Darüber hinaus ist die Reduktion dieser Aldehyde durch Natriumborhydrid beschrieben, wobei ein 1-Azabicyclo[2.2.2]octan als Pseudoenantiomerengemisch erhalten wird.

J. Chem. Soc. Perkin Trans. I 1995, 641-644 schlägt die Herstellung 3-substituierter Chinuklidine durch intramolekulare N-Alkylierung geeigneter Piperidinderivate vor. Zum Erhalt enantiomerenreiner 1-Azabicyclo[2.2.2]octane wurden bisher entsprechende chirale Piperidine als Vorläufer hergestellt und dann beispielsweise entsprechend der folgenden Reaktionsgleichung zyklisiert. 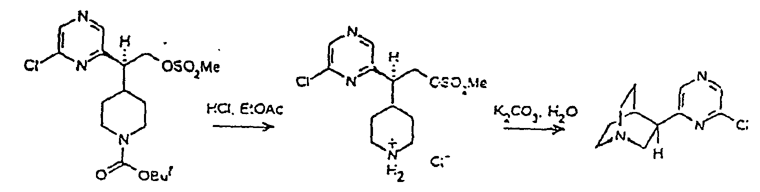 Dieses Verfahren ist jedoch nachteilig wegen des Erfordernisses, zuerst chirale Vorläuferverbindungen herzustellen.

Der Erfindung liegt somit die Aufgabe zugrunde, substituierte Chinuklidinderivate in enantiomerenreiner Form unter Umgehung der Herstellung chiraler Vorläuferverbindungen herzustellen.

Gelöst wird diese Aufgabe durch das Verfahren nach Anspruch 1.

Das erfindungsgemäße Verfahren erlaubt den direkten Zugriff auf 4 der 5 chiralen Zentren des Naturstoffs, d.h. die Konfiguration der Brückenkopfatome N(1) und C(4) sowie der Verzweigungsstellen an C(3) und C(8) des Naturstoffs (traditionelle Atomnumerierung nach Rabe) bleibt im abgespaltenen 1-Azabicyclo[2.2.2]octan erhalten [N(1), C(4), C(5) und C(2)] und ist definiert. Eine de novo Synthese des 1-Azabicyclo[2.2.2]octankäfigs aus enantiomerenreinen Vorläufern erübrigt sich.

In dem erfindungsgemäßen Verfahren erfolgt die Reduktion der Chinaalkaloide durch komplexe Metallhydride. Bevorzugt werden LiAlH₄ sowie dessen Alkoxyderivate LiAlH₂(OR)₂ mit R=C₁ bis C₆ eingesetzt. Die gleichzeitige Oxidation wird durch Luftzutritt an das Reaktionsgemisch bewirkt. Die Reaktion erfolgt in wasserfreien organischen Lösungsmitteln, in denen Reduktionen mit komplexen Metallhydriden üblicherweise durchgeführt werden. Bevorzugt werden wasserfreie Ether, insbesondere Tetrahydrofuran, eingesetzt.

Enantiomerenreine 1-Azabicyclo[2.2.2]-octane besitzen die allgemeinen Formeln 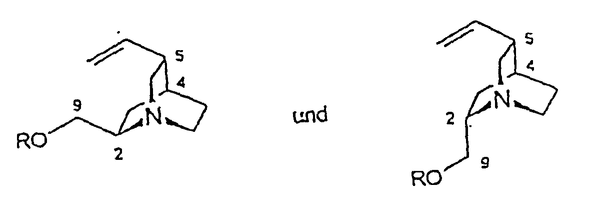 worin R Wasserstoff, eine geradkettige oder verzweigte Alkoxycarbonylgruppe mit 1 bis 12 Kohlenstoffatomen, eine geradkettige oder verzweigte Alkylgruppe mit 1 bis 12 Kohlenstoffatomen, alkylierte Silylgruppe, wie tert.-Butyldimethylsilyl oder einen substituierten Phenyl- oder Benzylrest bedeutet.

Erfindungsgemäß erhaltene Verbindungen sind beispielsweise
- (2R, 5R) -2-(t-Butoxycarbonyloxymethyl) -5-ethenyl-1-azabicyclo[2.2.2]octan (2b),
- (2R, 5R, 9R/S) -2-(t-Butoxycarbonyloxymethyl)-9-deuterio-5-ethenyl-1-azabicyclo[2.2.2]-octan (4a, 4b),
- (2S, 5R) -2- (t-Butoxycarbonyloxymethyl)-5-ethenyl-1-azabicyclo[2.2.2]octan (6b),
- (2S, 5R, 9R/S) -2- (t-Butoxycarbonyloxymethyl)-9-deuterio-5-ethenyl-1-azabicyclo[2.2.2]-octan (7a, 7b).

Diese Verbindungen sind wertvolle Zwischenverbindungen für die Herstellung von Arzneistoffen wie Muskarinagonisten.

Ein bevorzugter Syntheseweg des erfindungsgemäßen Verfahrens ist nachfolgend grafisch dargestellt.

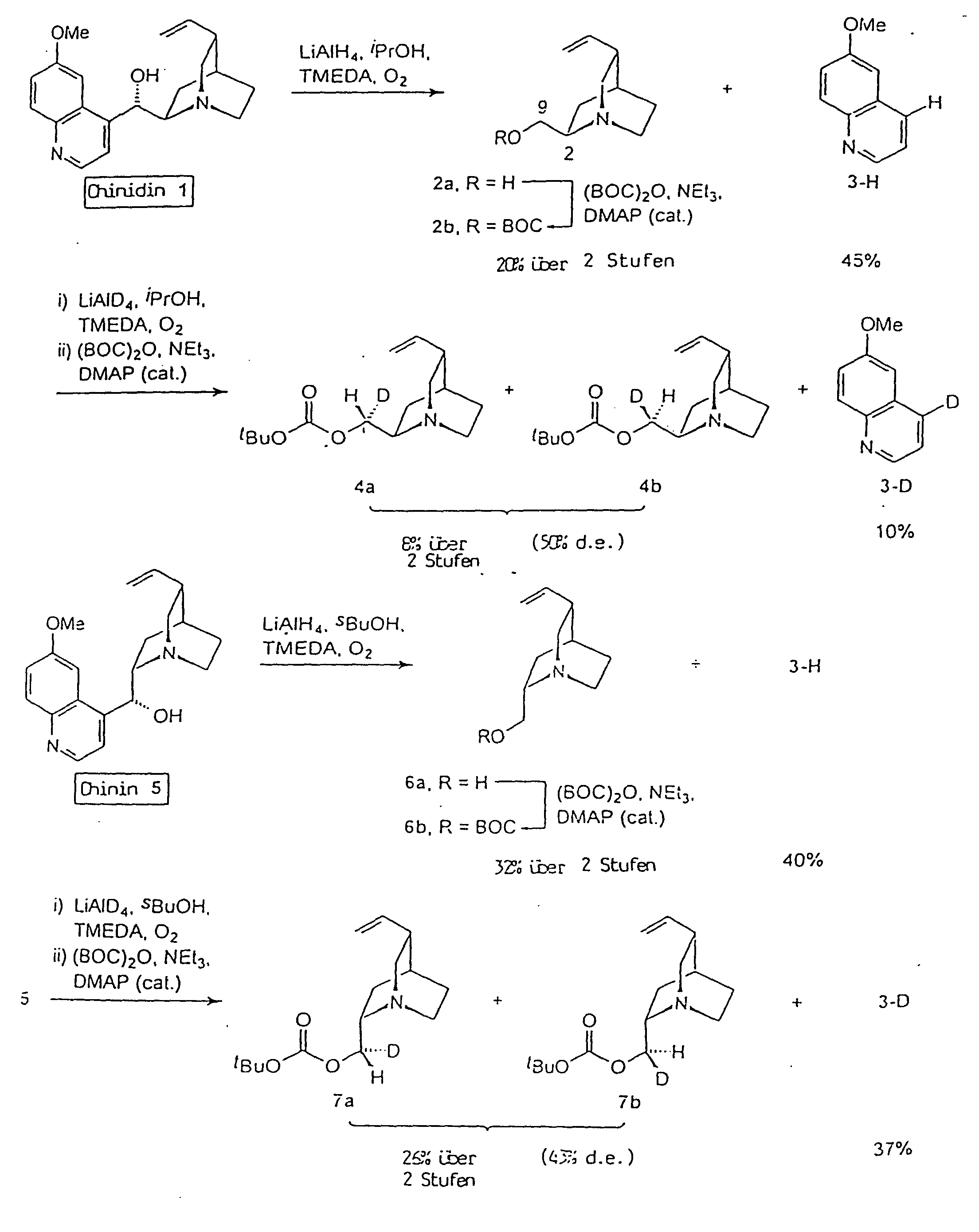

### Beispiele

Ein 50 ml Rundkolben wird mit trockenem THF (14 ml) und LiAlH₄ (304 mg, 8 mH) oder LiAlD₄ (336 mg, 8mM) gefüllt. Ein sekundärer Alkohol, sek. Butanol, und Tetramethylethylendiamin (TMEDA) (0,60 ml, 4mM) werden bei 0 °C hinzugegeben. Ein Chinaalkaloid (649 mg, 2 mM) wird in mehreren Anteilen bei 0 °C hinzugegeben. Das Gemisch wird 4 Tage bei Raumtemperatur unter Luftzutritt (Trockenrohr) gerührt und durch aufeinanderfolgende Zugabe von Ethylacetat (0,29 ml), Wasser (0,3 ml) und 2N NaOH (0,3 ml) aufgearbeitet. Der erhaltene Niederschlag wird abfiltriert und gründlich mit CH₂Cl₂ gewaschen. Nach Entfernung der Lösungsmittel wird der Rückstand in CH₂Cl₂ aufgenommen und getrocknet (beispielsweise über Natriumsulfat).

CH₂Cl₂ wird entfernt und das erhaltene viskose gelbe Öl wird im Vakuum getrocknet und in trockenem CH₂Cl₂ (4 ml) aufgenommen. Die Lösung wird mit Triethylamin (0,84 ml, 6 mM), bis-tert.-Butyldicarbonat (1,38 ml, 6 mM) und Dimethylaminopyridin in katalytischen Mengen behandelt und bei Raumtemperatur über Nacht gerührt. Das Rohgemisch der Produkte wird an Kieselgel adsorbiert und mit einem Gemisch aus Diethylether/Petrolether (1:2) als Elutionsmittel chromatographiert. Die isolierten Produkte werden als blaßgelbliche Öle gewonnen.

### Experimentelle Daten

(2R,5R)-2-(*t*-Butoxycarbonyloxymethyl)-5-ethenyl-1-azabicyclo[2.2.2]octan (**2b**) und 6-Methoxychinolin (**3-H**). Reduktionsmittel: LiAlH₄, sekundärer Alkohol: *i*-PrOH (1.07 mL, 14 mmol), Chinaalkaloid: Chinidin, Ausbeute: 107 mg (20%) **2b** und 143 mg (45%) **3-H**.

Daten für **2b**: [α]_{D}²⁰= +91.4° (c=1.25, CHCl₃). IR (kap. Film): ν = 793, 864, 913, 1097, 1164, 1256, 1278, 1370, 1456, 1741, 2873, 2938, 2978, 3077 cm⁻¹. ¹H NMR (200 MHz, CDCl₃): δ = 1.20-1.36 (m, 1H-3), 1.40-1.68 (m, 3H-8,8,3), 1.47 (s, 9H-*t*-BuO), 1.71-1.80 (m, 1H-4), 2.17-2.34 (m, 1H-5), 2.71 (ddd, ²J = 14 Hz, ³J = 7 Hz, ⁴J = 2 Hz, 1H-6), 2.78-3.15 (m, 4H-7,7,6,2), 3.94 (dd, ²J = 11 Hz, ³J = 5 Hz, 1H-9), 4.08 (dd, ²J = 11 Hz, ³J = 9 Hz, 1H-9), 5.02 (ddd, ²J = 1 Hz, ³Jₜᵣₐₙₛ = 17 Hz, ⁴J = 1 Hz, 1H-11), 5.04 (ddd, ²J = 1 Hz, ³J_{cis} = 11 Hz, ⁴J = 1 Hz, 1H-11), 5.74-5.95 (m, 1H-10). ¹³C NMR (50 MHz, APT, CDCl₃): δ = 24.06 (+, C-3), 26.72 (+, C-8), 27.47 (-, C-4), 27.75 (-, 3Me), 39.81 (-, C-5), 47.35 (+, C-7), 48.99 (+, C-6), 54.31 (-, C-2), 66.92 (+, C-9), 81.90 (+, 4°C), 114.61 (+, C-11), 140.31 (-, C-10), 153.72 (+, C=O). MS-MAT (RT): m/z (%): 267 (33) [M⁺], 211 (14), 194 (18), 170 (16), 159 (13), 150 (57), 136 (100), 116 (14), 102 (21). HRMS berechnet für C₁₅H₂₅NO₃: 267.1834, gefunden 267.1835.

Daten für **3-H**: IR (kap. Film): ν = 836, 848, 1027, 1038, 1128, 1163, 1231, 1270, 1500, 1597, 1624, 1698, 1741, 2934, 2975, 3377 cm⁻¹. ¹H NMR (200 MHz, CDCl₃): δ = 3.85 (s, 3H-MeO), 6.98 (d, ⁴J = 3 Hz, 1H-5), 7.28 (dd, ³J = 8,4 Hz, 1H-3), 7.34 (dd, ³J = 9 Hz, ⁴J = 3 Hz, 1H-7), 7.96 (dd, ³J = 8 Hz, ⁴J = 2 Hz, 1H-4), 8.00 (d, ³J = 9 Hz, 1H-8), 8.74 (dd, ³J = 4 Hz, ⁴J = 2 Hz, 1H-2). ¹³C NMR (50 MHz, APT, CDCl₃): δ = 55.41 (-, MeO), 105.05 (-, C-7), 121.30 (-, C-3), 122.23 (-, C-5), 129.25 (+, C-9), 130.76 (-, C-8), 134.71 (-, C-4), 144.36 (+, C-10), 147.85 (-, C-2), 157.64 (+, C-6). MS-MAT (RT): m/z (%): 159 (100) [M⁺], 144 (7), 129 (13), 116 (52), 101 (3), 89 (16), 80 (3), 75 (4). HRMS berechnet für C₁₀H₉NO: 159.0684, gefunden 159.0683.

(2R,5R,9R/S)-2-(*t*-Butoxycarbonyloxymethyl)-9-deuterio-5-ethenyl-1-azabicyclo[2.2.2]octan (**4a+4b**) und 4-Deuterio-6-methoxychinolin(**3-D**). Reduktionsmittel: LiAlH₄, sekundärer Alkohol: *i*-PrOH (1.07 mL, 14 mmol), Chinaalkaloid: Chinidin, Ausbeute: 43 mg (8%) of **4a+4b** (50% d.e.) und 32 mg (10%) of **3-D**.

Daten für **4a+4b**: IR (kap. Film): ν = 793, 859, 913, 990, 1165, 1256, 1278, 1370, 1456, 1741, 2873, 2937, 2978, 3077 cm⁻¹. ¹H NMR (200 MHz, CDCl₃): δ = 1.20-1.36 (m, 1H-3), 1.40-1.68 (m, 3H-8,8,3), 1.47 (s, 9H-t-BuO), 1.71-1.80 (m, 1H-4), 2.17-2.34 (m, 1H-5), 2.71 (ddd, ²J = 14 Hz, ³J = 7 Hz, ⁴J = 2 Hz, 1H-6), 2.78-3.15 (m, 4H-7,7,6,2), 3.93 (bd, ³J = 5 Hz, 1H-9), 4.06 (bd, ³J = 9 Hz, 1H-9), 5.02 (ddd, ²J = 1 Hz, ³Jₜᵣₐₙₛ = 17 Hz, ⁴J = 1 Hz, 1H-11), 5.04 (ddd, ²J = 1 Hz, ³J_{cis} = 11 Hz, ⁴J = 1 Hz, 1H-11), 5.74-5.95 (m, 1H-10). ¹³C NMR (50 MHz, BB, CDCl₃): δ = 24.16 (C-3), 26.81 (C-8), 27.59 (C-4), 27.82 (3Me), 39.87 (C-5), 47.54 (C-7), 49.09 (C-6), 54.37 (C-2) 66.34, 66.79, 67.24 (C-9), 81.93 (4°C), 114.63 (C-11), 140.39 (C-10), 153.80 (C=O). MS-MAT (RT): m/z (%): 268 (27) [M⁺], 212 (17), 195 (17), 171 (16), 151 (57), 136 (100), 117 (11). HRMS berechnet für C₁₅H₂₄DNO₃: 268.1896, gefunden 268.1896.

Daten für **3-D**: IR (kap.Film): ν = 710, 833, 875, 1029, 1121, 1152, 1230, 1262, 1300, 1373, 1430, 1468, 1500, 1584, 1620, 1747, 1936, 2938, 2960, 3392 cm⁻¹. ¹H NMR (200 MHz, CDCl₃): δ = 3.89 (s, 3H-MeO), 7.03 (d, ⁴J = 3 Hz, 1H-5), 7.32 (d, ³J = 4 Hz, 1H-3), 7.36 (dd, ³J = 9 Hz, ⁴J = 3 Hz, 1H-7), 7.99 (d, ³J = 9 Hz, 1H-8), 8.75 (d, ³J = 4 Hz, 1H-2). ¹³C NMR (50 MHz, BB, CDCl₃): δ = 54.46 (MeO), 104.13 (C-7), 120.18 (C-3), 121.20 (C-5), 128.23 (C-9), 129.82 (C-8), 132,89, 133.38, 133.87 (C-4), 143.45 (C-10), 146.88 (C-2), 156.75 (C-6). MS-MAT (RT): m/z (%): 160.(100) [M⁺], 145 (6), 130 (11), 117 (50), 103 (3), 90 (12), 80 (4), 76 (2). HRMS berechnet für C₁₀H₈DNO: 160.0746, gefunden 160.0745.

(2S,5R)-2-(*t*-Butoxycarbonyloxymethyl)-5-ethenyl-1-azabicyclo[2.2.2]octan (**6b**) and 6-Methoxychinolin (**3-H**). Reduktionsmittel: LiAlH₄, sekundärer Alkohol: *s*-BuOH (1.28 mL, 14 mmol), Chinaalkaloid: Chinin, Ausbeute: 171 mg (32%) **6b** und 127 mg (40%) **3-H**.

Daten für **6b**: [α]_{D}²⁰= +29.4° (c=1.38, CHCl₃). IR (kap. Film): ν = 794, 865, 912, 1097, 1165, 1256, 1278, 1370, 1396, 1455, 1475, 1741, 2865, 2937, 2978, 3077 cm⁻¹. ¹H NMR (200 MHz, CDCl₃): δ = 0.85-1.02 (m, 1H-3), 1.41-1.61 (m, 2H-8), 1.47 (s, 9H-t-BuO), 1.70-1.80 (m, 1H-4), 1.80-1.97 (m, 1H-3), 2.21-2.39 (m, 1H-5), 2.60-2.80 (m, 2H-7,6), 2.90-3.16 (m, 2H-7,2), 3.19 (dd, ²J = 13 Hz, ³J = 10 Hz, 1H-6), 3.97 (dd, ²J = 11 Hz, ³J = 6 Hz, 1H-9), 4.08 (dd, ²J = 11 Hz, ³J = 9 Hz, 1H-9) 5.03 (ddd, ²J = 1 Hz, ³J_{cis} = 11 Hz, ⁴J = 1 Hz, 1H-11), 5.04 (ddd, ²J = 1 Hz, ³Jₜᵣₐₙₛ = 17 Hz, ⁴J = 1 Hz, 1H-11) 5.77-5.98 (m, 1H-10). ¹³C NMR (75 MHz, APT, CDCl₃): δ = 24.91 (+, C-3), 27.35 (-, C-4), 27.72 (-, 3Me), 27.79 (+, C-8), 39.71 (-, C-5), 40.96 (+, C-7), 54.42 (-, C-2), 55.73 (+, C-6), 67.60 (+, C-9), 81.90 (+, 4°C), 114.29 (+, C-11), 141.73 (-, C-10), 153.69 (+, C=O). MS-MAT (RT): m/z (%): 267 (37) [M⁺], 252 (17), 226 (3), 211 (28), 194 (34), 182 (7), 170 (32), 159 (48), 150 (51), 144 (28), 136 (100), 129 (30), 116 (46), 108 (29), 95 (30), 89 (30), 79 (35). HRMS berechnet für C₁₅H₂₅NO₃: 267.1834, gefunden 267.1834.

Daten für **3-H**: siehe oben.

(2S,5R,9R/S)-2-(t-Butoxycarbonyloxymethyl)-9-deuterio-5-ethenyl-1-azabicyclo[2.2.2]octan (**7a+7b**) und 4-Deuterio-6-methoxychinolin(**3-D**). Reduktionsmittel: LiAlH₄, sekundärer Alkohol: *s*-BuOH (0.55 mL, 6 mmol), Chinaalkaloid: Chinin, Ausbeute: 140 mg (26%) **7a+7b** (43% d.e.) und 119 mg (37%) **3-D**.

Daten für **7a+7b**: IR (kap. Film): ν = 794, 857, 913, 1049, 1165, 1256, 1278, 1370, 1395, 1456, 1741, 2867, 2937, 2978, 3078 cm⁻¹. ¹H NMR (200 MHz, CDCl₃): δ = 0.85-1.02 (m, 1H-3), 1.41-1.61 (m, 2H-8), 1.47 (s, 9H-t-BuO), 1.70-1.80 (m, 1H-4), 1.80-1.97 (m, 1H-3), 2.21-2.39 (m, 1H-5), 2.60-2.80 (m, 2H-7,6), 2.90-3.16 (m, 2H-7,2), 3.19 (dd, ²J = 13 Hz, ³J = 10 Hz, 1H-6), 3.96 (bd, ³J = 6 Hz, 1H-9), 4.05 (bd, ³J = 9 Hz, 1H-9) 5.03 (ddd, ²J = 1 Hz, ³J_{cis} = 11 Hz, ⁴J = 1 Hz, 1H-11), 5.04 (ddd, ²J = 1 Hz, ³Jₜᵣₐₙₛ = 17 Hz, ⁴J = 1 Hz, 1H-11) 5.77-5.98 (m, 1H-10). ¹³C NMR (50 MHz, BB, CDCl₃): δ = 24.84 (C-3), 27.39 (C-4), 27.69 (C-8), 27.79 (3Me), 39.60 (C-5), 41.02 (C-7), 54,42 (C-2), 55.56 (C-6), 66.80, 67.25, 67.70 (C-9), 81.98 (4°C), 114.50 (C-11), 141.56 (C-10), 153.71 (C=O). MS-MAT (RT): m/z (%): 268 (27) [M⁺], 258 (2), 227 (2), 212 (9), 195 (13), 180 (3), 171 (11), 160 (18), 151 (27), 145 (3), 136 (100), 130 (5), 117 (11), 110 (9), 96 (9), 82 (17), 73 (17). HRMS berechnet für C₁₅H₂₄DNO₃: 268.1896, gefunden 268.1896.

Daten für **3-D**: siehe oben.

## Patentansprüche

1. Verfahren zur Herstellung eantiomerenreiner 1-Azablcyclo[2.2.2]octane der allgemeinen Formel 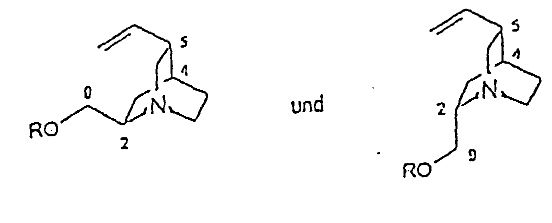 worin R Wasserstoff, eine geradkettige oder verzweigte Alkoxycarbonylgruppe mit 1-12 Kohlenstoffatomen, eine geradkettige oder verzweigte Alkylgruppe mit 1-12 Kohlenstoffatomen, eine alkylierte Silylgruppe nämlich tert.-Butyldimethylsilyl oder einen substituierten Phenyl- oder Benzylrest bedeutet,
dadurch gekennzeichnet, daß man ein Chinaalkaloid mit einem komplexen Metallhydrid unter gleichzeitiger Oxidation durch Luftzutritt in einem Ether oder Tetrahydrofuran behandelt, wobei das Chinaalkaloid in einen Chinolinabkömmling und ein enantiomerenreines 1-Azabicyclo[2.2.2]octan gespalten wird und für den Fall R ungleich H der Wasserstoff aus der OH-Gruppe des primär erhaltenen 1-Azabicyclo[2.2.2]octans mit einer geradkettigen oder verzweigten Alkoxycarbonylgruppe mit 1-12 Kohlenstoffatomen, einer geradkettigen oder verzweigten Alkylgruppe mit 1-12 Kohlenstoffatomen, einer alkylierten Silylgruppe, nämlich tert-Butyldimethylsilyl, oder einem substituierten Phenyl- oder Benzylrest substituiert wird.

2. Verfahren nach Anspruch 1, dadurch gekennzeichnet, daß als komplexes Metallhydrid Lithiumaluminiumhydrid oder ein Lithiumalkoxyaluminiumhydrid verwendet wird.

3. Enantiomerenreine 1-Azabicyclo[2.2.2]octane der allgemeinen Formel 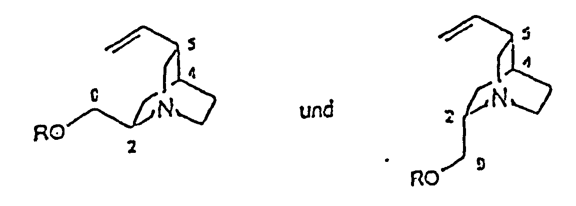 worin R Wasserstoff, eine geradkettige oder verzweigte Alkoxycarbonylgruppe mit 1-12 Kohlenstoffatomen, eine geradkettige oder verzweigte Alkylgruppe mit 1-12 Kohlenstoffatomen, eine alkylierte Silylgruppe nämlich tert.-Butyldimethylsilyl oder einen substituierten Phenyl- oder Benzylrest bedeutet.

4. Verwendung der enantiomerenreinen 1-Azabicyclo[2.2.2]octane nach Anspruch 3 als Zwischenverbindungen für die Herstellung von Arzneistoffen.

## Claims

1. Process for the preparation of enantiomerically pure 1-azabicyclo[2.2.2]octanes of the general formula 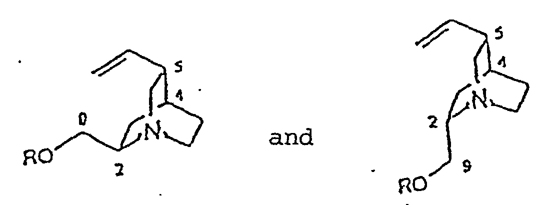 wherein R represents hydrogen, a straight-chained or branched alkoxycarbonyl group having from 1 to 12 carbon atoms, a straight-chained or branched alkyl group having from 1 to 12 carbon atoms, an alkylated silyl group, namely tert-butyldimethylsilyl, or a substituted phenyl or benzyl radical,
characterised in that a cinchona alkaloid is treated in an ether or tetrahydrofuran with a complex metal hydride with simultaneous oxidation by admission of air, the cinchona alkaloid being cleaved into a quinoline derivative and an enantiomerically pure 1-azabicyclo[2.2.2]octane, and, in the case where R is other than H, the hydrogen from the OH group of the 1-azabicyclo[2.2.2]octane obtained as primary product is substituted by a straight-chained or branched alkoxycarbonyl group having from 1 to 12 carbon atoms, a straight-chained or branched alkyl group having from 1 to 12 carbon atoms, an alkylated silyl group, namely tertbutyldimethylsilyl, or by a substituted phenyl or benzyl radical.

2. Process according to claim 1, characterised in that lithium aluminium hydride or a lithium alkoxyaluminium hydride is used as the complex metal hydride.

3. Enantiomerically pure 1-azabicyclo[2.2.2]octanes of the general formula 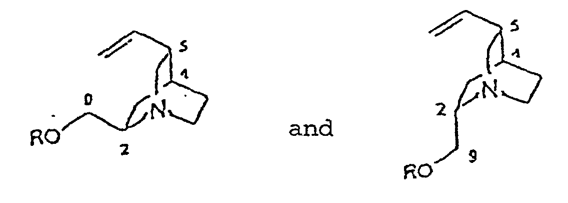 wherein R represents hydrogen, a straight-chained or branched alkoxycarbonyl group having from 1 to 12 carbon atoms, a straight-chained or branched alkyl group having from 1 to 12 carbon atoms, an alkylated silyl group, namely tert-butyldimethylsilyl, or a substituted phenyl or benzyl radical.

4. Use of the enantiomerically pure 1-azabicyclo[2.2.2]octanes according to claim 3 as intermediate compound for the preparation of medicaments.

## Revendications

1. Procédé de fabrication d'énantiomères purs de 1-azabicyclo[2.2.2]octane de formule générale 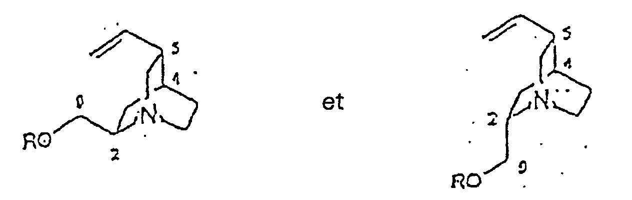 dans laquelle R est un hydrogène, un groupement alkoxycarbonyle linéaire ou ramifié avec 1-12 atomes de carbone, un groupement alkyle linéaire ou ramifié avec 1-12 atomes de carbone, un groupement silyle alkyl, notamment le tertio-butyldiméthylsilyle, ou un résidu phényle ou benzyle substitué,
caractérisé en ce que l'on soumet un quinaalkaloïde avec un hydrure métallique complexé sous oxydation simultanée sous arrivée d'air et dans un éther ou dans du tétrahydrofurane, de sorte que le quinaakaloïde se décompose en un dérivé de quinoline et en un énantiomère pur de 1-azabicyclo[2.2.2]octane et, dans le cas où R est différent de H, l'hydrogène du groupement OH de l'1-azabicyclo[2.2.2]octane obtenu précédemment est substitué par un groupement alkoxycarbonyle linéaire ou ramifié avec 1-12 atomes de carbone, un groupement alkyle linéaire ou ramifié avec 1-12 atomes de carbone, un groupement silyle alkyl, notamment le tertio-butyldiméthylsilyle, ou un résidu phényle ou benzyle substitué.

2. Procédé selon la revendication 1, caractérisé en ce que l'on utilise comme hydrure métallique complexé un hydrure de lithiumaluminium ou un hydrure de lithiumalkoxyaluminium.

3. Enantiomères purs de 1-azabicyclo[2.2.2]octane de formule générale 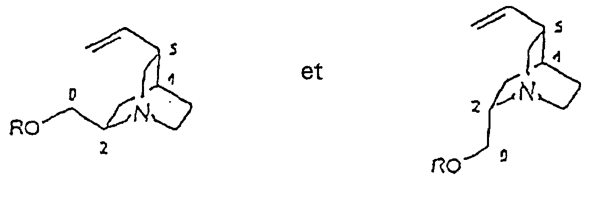 dans laquelle R est un hydrogène, un groupement alkoxycarbonyle linéaire ou ramifié avec 1-12 atomes de carbone, un groupement alkyle linéaire ou ramifié avec 1-12 atomes de carbone, un groupement silyle alkyl, notamment le tertio-butyldiméthylsilyle, ou un résidu phényle ou benzyle substitué.

4. Utilisation des énantiomères purs de 1-azabicyclo[2.2.2]octane selon la revendication 3 comme composé intermédiaire pour la fabrication de médicaments.
